# EUROPEAN PATENT APPLICATION

(11) **EP 4 837 634 A2**
(43) Date of publication of application: **11.11.2026**
(21) Application number: 26192777.6
(22) Date of filing: 14.03.2014
(51) Int. Cl.: A61P 31/12

(54) **COMBINATION OF TWO ANTIVIRALS FOR TREATING HEPATITIS C**

(30) Priority: 14.03.2013 US 201361783376 P
(62) Document number(s) of the earlier application(s) in accordance with Art. 76 EPC: 20183791.1 / 3 766 495; 17165207.6 / 3 213 750; 14719977.2 / 2 968 301
(71) Applicant: AbbVie Inc., North Chicago, IL 60064 (US)
(72) Inventor: BERNSTEIN, Barry M., Mequon WI, 53092 (US); DUTTA, Sandeep, Lincolnshire IL, 60069 (US); LIU, Wei, Mundelein IL, 60060 (US); PODSADECKI, Thomas J., Northbrook IL, 60062 (US); CAMPBELL, Andrew L., Lake Forest IL, 60045 (US); MENON, Rajeev M., Buffalo Grove IL, 60089 (US); LIN, Chih-Wei, Vernon Hills IL, 60061 (US); WANG, Tianli, Lincolnshire IL, 60069-3220 (US); AWNI, Walid M., Green Oaks IL, 60048 (US); MENSING, Sven, 68305 Mannheim (DE)
(74) Representative: Mewburn Ellis LLP

(57) **Abstract**

The present invention features interferon- and ribavirin-free therapies for the treatment of HCV. Preferably, the treatment is over a shorter duration of treatment, such as no more than 12 weeks. In one aspect, the treatment comprises administering at least two direct acting antiviral agents without interferon and ribavirin to a subject with HCV infection, wherein the treatment lasts for 12 weeks, and said at least two direct acting antiviral agents comprise (a) Compound 1 or a pharmaceutically acceptable salt thereof and (b) Compound 2 or a pharmaceutically acceptable salt thereof.

## Description

This application claims the benefit of U.S. Provisional Application No. 61/783,376, filed March 14, 2013, which is incorporated herein by reference in its entirety.

### FIELD OF THE INVENTION

The present invention relates to interferon-free and ribavirin-free treatment for hepatitis C virus (HCV).

### BACKGROUND OF THE INVENTION

The HCV is an RNA virus belonging to the Hepacivirus genus in the Flaviviridae family. The enveloped HCV virion contains a positive stranded RNA genome encoding all known virus-specific proteins in a single, uninterrupted, open reading frame. The open reading frame comprises approximately 9500 nucleotides and encodes a single large polyprotein of about 3000 amino acids. The polyprotein comprises a core protein, envelope proteins E1 and E2, a membrane bound protein p7, and the non-structural proteins NS2, NS3, NS4A, NS4B, NS5A and NS5B.

Chronic HCV infection is associated with progressive liver pathology, including cirrhosis and hepatocellular carcinoma. Chronic hepatitis C may be treated with peginterferon-alpha in combination with ribavirin. Substantial limitations to efficacy and tolerability remain as many users suffer from side effects, and viral elimination from the body is often incomplete. Therefore, there is a need for new therapies to treat HCV infection.

### BRIEF SUMMARY OF THE INVENTION

One aspect of the present invention features methods for treating HCV infection in a subject in need of such treatment. The methods comprise administering at least two direct acting antiviral agents (DAAs) to the subject for a duration of no more than 12 weeks, or for another duration as set forth herein. The at least two DAAs comprise Compound 1 (or a pharmaceutically acceptable salt thereof) and Compound 2 (or a pharmaceutically acceptable salt thereof). Preferably, the duration of the treatment is 12 weeks. The duration of the treatment can also be, for example, no more than 8 weeks. Preferably, the two or more DAAs are administered in amounts effective to provide a sustained virological response (SVR) or achieve another desired measure of effectiveness in the subject. The subject is not administered ribavirin during the treatment regimen. The subject is also not administered interferon during the treatment regimen. Put another way, the methods exclude the administration of interferon or ribavirin to the subject, thereby avoiding the side effects associated with interferon and ribavirin.

Another aspect of the present invention features methods for treating a population of subjects having HCV infection. The methods comprise administering at least two DAAs to the subjects for a duration of no more than 12 weeks. The at least two DAAs comprise Compound 1 (or a pharmaceutically acceptable salt thereof) and Compound 2 (or a pharmaceutically acceptable salt thereof). Preferably, the at least two DAAs are administered to the subjects in amounts effective to result in SVR or another measure of effectiveness in at least about 70% of the population, preferably at least about 80% of the population, or more preferably at least about 90% of the population.

In any method described herein, the at least two DAAs comprise (a) Compound 1 or a pharmaceutically acceptable salt thereof, and (b) Compound 2 or a pharmaceutically acceptable salt thereof. The at least two DAAs can also optionally comprise another anti-HCV agent. The other optional anti-HCV agent can be selected from protease inhibitors, nucleoside or nucleotide polymerase inhibitors, non-nucleoside polymerase inhibitors, NS3B inhibitors, NS4A inhibitors, NS5A inhibitors, NS5B inhibitors, cyclophilin inhibitors, or combinations thereof. For example, in some embodiments, the DAAs used in a method of the present invention comprise or consist of (a) Compound 1 or a pharmaceutically acceptable salt thereof, and (b) Compound 2 or a pharmaceutically acceptable salt thereof. For another example, the DAAs used in a method of the present invention comprise or consist of (a) Compound 1 or a pharmaceutically acceptable salt thereof, (b) Compound 2 or a pharmaceutically acceptable salt thereof, and (c) a HCV polymerase inhibitor, wherein said HCV polymerase inhibitor can be a nucleotide or nucleoside polymerase inhibitor or a non-nucleoside or non-nucleotide polymerase inhibitor.

Non-limiting examples of the other optional antic-HCV agent include PSI-7977 (sofosbuvir), PSI-938, BMS-790052 (daclatasvir), BMS-650032 (asunaprevir), BMS-791325, GS-5885 (ledipasvir), GS-9451 (tegobuvir), GS-9190, GS-9256, BI-201335, BI-27127, telaprevir, VX-222, TMC-435 (simepravir), MK-5172, MK-7009 (vaniprevir ), danoprevir, R7128 (mericitabine), and any combination thereof.

In any method described herein, the DAAs can be administered in any effective dosing schemes and/or frequencies; for example, they can each be administered daily. Each DAA can be administered either separately or in combination, and each DAA can be administered once a day, twice a day, or three times a day. Preferably, Compound 1 (or a pharmaceutically acceptable salt thereof) and Compound 2 (or a pharmaceutically acceptable salt thereof) are administered once daily.

Preferably, Compound 1 (or a pharmaceutically acceptable salt thereof) is administered from 100 mg to 600 mg once daily, and Compound 2 (or a pharmaceutically acceptable salt thereof) is administered from 50 to 500 mg once daily. More preferably, Compound 1 (or a pharmaceutically acceptable salt thereof) is administered from 200 mg to 600 mg once daily, and Compound 2 (or a pharmaceutically acceptable salt thereof) is administered from 100 to 500 mg once daily. Highly preferably, Compound 1 (or a pharmaceutically acceptable salt thereof) is administered from 400 mg to 600 mg once daily, and Compound 2 (or a pharmaceutically acceptable salt thereof) is administered from 100 to 500 mg once daily. For instance, Compound 1 (or a pharmaceutically acceptable salt thereof) can be administered 400 mg once daily, and Compound 2 (or a pharmaceutically acceptable salt thereof) is administered 120 mg once daily. For another instance, Compound 1 (or a pharmaceutically acceptable salt thereof) can be administered 400 mg once daily, and Compound 2 (or a pharmaceutically acceptable salt thereof) can be administered 240 mg once daily.

In yet another aspect, the present invention features a combination of Compound 1 (or a pharmaceutically acceptable salt thereof) and Compound 2 (or a pharmaceutically acceptable salt thereof) for use to treat HCV infection. The treatment comprises administering the DAAs to a subject infected with HCV. The duration of the treatment regimen is no more than twelve weeks (e.g., the duration being 12 weeks; or the duration being 11, 10, 9, 8, 7, 6, 5, 4, or 3 weeks). Preferably, the duration of the treatment regimen is twelve weeks. The duration of the treatment can also last, for example, no more than eight weeks (e.g., the duration being 8 weeks; or the duration being 7, 6, 5, 4, or 3 weeks). The treatment does not include administering interferon or ribavirin. Compound 1 (or the salt thereof) and Compound 2 (or the salt thereof) can be administered concurrently or sequentially. Preferably, Compound 1 (or the salt thereof) and Compound 2 (or the salt thereof) can be administered once daily. As a non-limiting example, the patient being treated is infected with HCV genotype 1, such as genotype 1a or 1b. As another non-limiting example, the patient is infected with HCV genotype 2. As another non-limiting example, the patient is infected with HCV genotype 3. As another non-limiting example, the patient is infected with HCV genotype 4. As another non-limiting example, the patient is infected with HCV genotype 5. As another non-limiting example, the patient is infected with HCV genotype 6. As yet another non-limiting example, the patient is a HCV-treatment naïve patient, a HCV-treatment experienced patient, an interferon non-responder (e.g., a null responder), or not a candidate for interferon treatment. As used in this application, the interferon non-responder patients include partial interferon responders and interferon rebound patients. *See* GUIDANCE FOR INDUSTRY - CHRONIC HEPATITIS C VIRUS INFECTION: DEVELOPING DIRECT-ACTING ANTIVIRAL AGENTS FOR TREATMENT (FDA, September 2010, draft guidance) for the definitions of naive, partial responder, responder relapser (i.e., rebound), and null responder patients. The interferon non-responder patients also include null responder patients. In one example of this aspect of the invention, the treatment lasts for 12 weeks, and the subject being treated is a naïve patient infected with HCV genotype 1. In another example, the treatment lasts for 11 weeks, and the subject being treated is a naïve patient infected with HCV genotype 1. In still another example, the treatment lasts for 10 weeks, and the subject being treated is a naïve patient infected with HCV genotype 1. In yet another example, the treatment lasts for 9 weeks, and the subject being treated is a naïve patient infected with HCV genotype 1. In yet another example, the treatment lasts for 8 weeks, and the subject being treated is a naïve patient infected with HCV genotype 1. In yet another example, the treatment lasts for 12 weeks, and the subject being treated is a naïve patient infected with HCV genotype 3. In another example, the treatment lasts for 11 weeks, and the subject being treated is a naïve patient infected with HCV genotype 3. In still another example, the treatment lasts for 10 weeks, and the subject being treated is a naïve patient infected with HCV genotype 3. In yet another example, the treatment lasts for 9 weeks, and the subject being treated is a naive patient infected with HCV genotype 3. In yet another example, the treatment lasts for 8 weeks, and the subject being treated is a naïve patient infected with HCV genotype 3. In yet another example, the treatment lasts for 12 weeks, and the subject being treated is a non-responder (e.g., a null responder) infected with HCV genotype 1. In another example, the treatment lasts for 11 weeks, and the subject being treated is a non-responder (e.g., a null responder) infected with HCV genotype 1. In still another example, the treatment lasts for 10 weeks, and the subject being treated is a non-responder (e.g., a null responder) infected with HCV genotype 1. In yet another example, the treatment lasts for 9 weeks, and the subject being treated is a non-responder (e.g., a null responder) infected with HCV genotype 1. In yet another example, the treatment lasts for 8 weeks, and the subject being treated is a non-responder (e.g., a null responder) infected with HCV genotype 1. In yet another example, the treatment lasts for 12 weeks, and the subject being treated is a non-responder (e.g., a null responder) infected with HCV genotype 3. In another example, the treatment lasts for 11 weeks, and the subject being treated is a non-responder (e.g., a null responder) infected with HCV genotype 3. In still another example, the treatment lasts for 10 weeks, and the subject being treated is a non-responder (e.g., a null responder) infected with HCV genotype 3. In yet another example, the treatment lasts for 9 weeks, and the subject being treated is a non-responder (e.g., a null responder) infected with HCV genotype 3. In yet another example, the treatment lasts for 8 weeks, and the subject being treated is a non-responder (e.g., a null responder) infected with HCV genotype 3.

A treatment regimen of the present invention generally constitutes a complete treatment regimen, i.e., no subsequent interferon-containing regimen is intended. Thus, a treatment or use described herein generally does not include any subsequent interferon-containing treatment. Preferably, a treatment or use described herein does not include any subsequent ribavirin-containing treatment.

Other features, objects, and advantages of the present invention are apparent in the detailed description that follows. It should be understood, however, that the detailed description, while indicating preferred embodiments of the invention, are given by way of illustration only, not limitation. Various changes and modifications within the scope of the invention will become apparent to those skilled in the art from the detailed description

### BRIEF DESCRIPTION OF THE DRAWINGS

The drawings are provided for illustration, not limitation.
Figure 1 shows the predicted median SVR percentages and 90% SVR confidence intervals for interferon/ribavirin-free, 2-DAA regimens comprising the use of Compound 1 (400 mg once daily) and Compound 2 (120 mg once daily) to treat genotype 1 naïve subjects.
Figure 2 illustrates the predicted median SVR percentages and 90% SVR confidence intervals for interferon/ribavirin-free, 2-DAA regimens comprising the use of Compound 1 (400 mg once daily) and Compound 2 (60 mg once daily) to treat genotype 1 naïve subjects.
Figure 3 depicts the predicted median SVR percentages and 90% SVR confidence intervals for interferon/ribavirin-free, 2-DAA regimens comprising the use of Compound 1 (600 mg once daily) and Compound 2 (480 mg once daily) to treat genotype 1 naïve subjects.
Figure 4 shows the predicted median SVR percentages and 90% SVR confidence intervals for interferon/ribavirin-free, 2-DAA regimens comprising the use of Compound 1 (400 mg once daily) and Compound 2 (120 mg once daily) to treat genotype 3 naïve subjects.
Figure 5 illustrates the predicted median SVR percentages and 90% SVR confidence intervals for interferon/ribavirin-free, 2-DAA regimens comprising the use of Compound 1 (400 mg once daily) and Compound 2 (60 mg once daily) to treat genotype 3 naïve subjects.
Figure 6 shows the predicted median SVR percentages and 90% SVR confidence intervals for interferon/ribavirin-free, 2-DAA regimens comprising the use of Compound 1 (600 mg once daily) and Compound 2 (480 mg once daily) to treat genotype 3 naïve subjects.
Figure 7 depict the synergistic effect of the combination of Compound 1 and Compound 2 on HCV inhibition *in vitro.*

### DETAILED DESCRIPTION OF THE INVENTION

The methods of the present invention include administering Compound 1 (or a pharmaceutically acceptable salt thereof) and Compound 2 (or a pharmaceutically acceptable salt thereof) to a subject in need thereof. Compound 1 has the following structure: 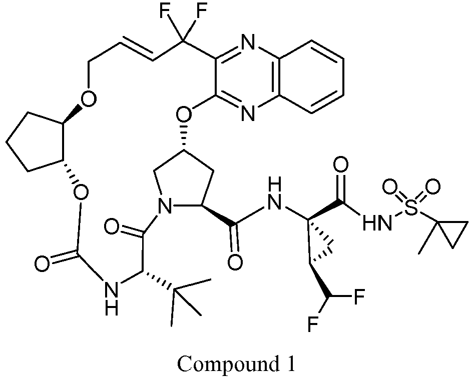 Compound 1 is a potent HCV protease inhibitor and is described in U.S. Patent Application Publication No. 2012/0070416.

Compound 2 has the following structure: 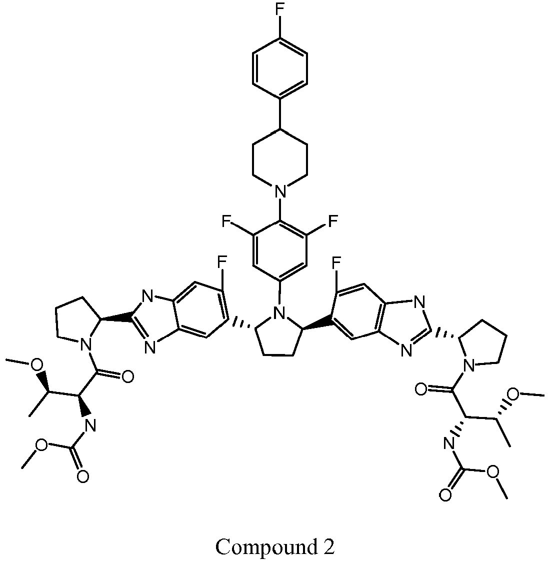 Compound 2 is a potent NS5A inhibitor and is described in U.S. Patent Application Publication No. 2012/0220562.

The current standard of care (SOC) for the treatment of HCV includes a course of treatment of interferon, e.g. pegylated interferon (e.g., pegylated interferon-alpha-2a or pegylated interferon-alpha-2b, such as PEGASYS by Roche, or PEG-INTRON by Schering-Plough) and the antiviral drug ribavirin (e.g., COPEGUS by Roche, REBETOL by Schering-Plough, or RIBASPHERE by Three Rivers Pharmaceuticals). The treatment often lasts for 24-48 weeks, depending on hepatitis C virus genotype. Other interferons include, but are not limited to, interferon-alpha-2a (e.g., Roferon-A by Roche), interferon-alpha-2b (e.g., Intron-A by Schering-Plough), and interferon alfacon-1 (consensus interferon) (e.g., Infergen by Valeant).

The interferon/ribavirin-based treatment may be physically demanding, and can lead to temporary disability in some cases. A substantial proportion of patients will experience a panoply of side effects ranging from a "flu-like" syndrome (the most common, experienced for a few days after the weekly injection of interferon) to severe adverse events including anemia, cardiovascular events and psychiatric problems such as suicide or suicidal ideation. The latter are exacerbated by the general physiological stress experienced by the patients. Ribavirin also has a number of side effects, including, anemia, high pill burden (e.g. 5-6 pills a day split BID) and teratogenicity restricting use in women of childbearing age.

The methods of the present invention provide effective treatment of HCV infection without the use of interferon or ribavirin and for a shorter period of time, for example and without limitation, a treatment duration of no more than twelve weeks, alternatively no more than eleven weeks, alternatively no more than ten weeks, alternatively no more than nine weeks, alternatively no more than eight weeks, alternatively no more than seven weeks, alternatively no more than six weeks, alternatively no more than five weeks, alternatively no more than four weeks, or alternatively, no more than three weeks.

In one aspect, the present invention features methods for treating HCV infection in a subject comprising administering at least two DAAs, in the absence of interferon and ribavirin, to the subject for a duration of no more than twelve weeks, alternatively no more than eight weeks. Put another way, the methods exclude interferon and ribavirin. The at least two DAAs comprise Compound 1 (or a pharmaceutically acceptable salt thereof) and Compound 2 (or a pharmaceutically acceptable salt thereof), which can be co-administered, or administered separately or independently, with the same or different dosing frequencies. Preferably, the at least two DAAs are administered once a day. They can also be administered, for example, twice a day or three times a day.

Various measures may be used to express the effectiveness of a method of the present invention. One such measure is SVR, which, as used herein, means that the virus is undetectable at the end of therapy and for at least 8 weeks after the end of therapy (SVR8); preferably, the virus is undetectable at the end of therapy and for at least 12 weeks after the end of therapy (SVR12); more preferably, the virus is undetectable at the end of therapy and for at least 16 weeks after the end of therapy (SVR16); and highly preferably, the virus is undetectable at the end of therapy and for at least 24 weeks after the end of therapy (SVR24). SVR24 is often considered as a functional definition of cure; and a high rate of SVR at less than 24 week post-treatment (e.g., SVR8 or SVR12) can be predictive of a high rate of SVR24.

In some embodiments, a treatment regimen of the invention comprises treating a population of subjects having HCV infection (e.g. treatment naïve subjects), and the regimen comprises administering at least two DAAs to the subjects for a duration of no more than 12 weeks, or for another duration disclosed herein, wherein the at least two DAAs comprise Compound 1 (or a pharmaceutically acceptable salt thereof) and Compound 2 (or a pharmaceutically acceptable salt thereof), and are administered to the subjects in amounts effective to provide an SVR (e.g., SVR12 or SVR24) in at least about 70% of the population, alternatively at least about 75% of the population, alternatively at least about 80% of the population, alternatively at least about 85% of the population, alternatively at least about 90% of the population, alternatively at least about 95% of the population, alternatively about 100% of the population. In some embodiments, a treatment regimen of the invention comprises treating a population of IFN experienced subjects (e.g., interferon non-responders) having HCV infection, and the method comprises administering at least two DAAs to the subjects for a duration of no more than 12 weeks, or for another duration disclosed herein, wherein the at least two DAAs comprise Compound 1 (or a pharmaceutically acceptable salt thereof) and Compound 2 (or a pharmaceutically acceptable salt thereof), and are administered to the subjects in amounts effective to provide an SVR (e.g., SVR12 or SVR24) in at least about 50% of the population, alternatively at least about 55% of the population, alternatively at least about 60% of the population, alternatively at least about 65% of the population, alternatively at least about 70% of the population, alternatively at least about 75% of the population, alternatively at least about 80% of the population, alternatively at least about 85% of the population, alternatively at least about 90% of the population, alternatively at least about 95% of the population, or alternatively about 100% of the population.

It was unexpected that an interferon-free and ribavirin-free treatment using a combination of Compound 1 (or a pharmaceutically acceptable salt thereof) and Compound 2 (or a pharmaceutically acceptable salt thereof), and for a duration of no more than 12 weeks, can achieve significant SVR.

Accordingly, in one aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 8 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 7 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 6 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 5 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 4 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 3 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 24 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 13 to 23 weeks (e.g., the duration of the treatment is selected from 13, 14, 15, 16, 17, 18, 19, 20, 21, 22 or 23 weeks) and does not include administration of any interferon or ribavirin. The DAAs can be administered at the same or different dosing frequencies. The patient being treated can be a treatment naïve patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 12 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir. As used in this application, a HCV polymerase inhibitor can be a nucleoside polymerase inhibitor, a nucleotide polymerase inhibitor, a non-nucleoside polymerase inhibitor, or a non-nucleotide polymerase inhibitor.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 11 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 10 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In yet another aspect, the present invention features a method of treating HCV infection, comprising administering to a patient in need thereof an effective amount of a combination of at least two DAAs, wherein said at least two DAAs comprise Compound 1 (or a pharmaceutically acceptable salt thereof) and Compound 2 (or a pharmaceutically acceptable salt thereof). The treatment lasts 9 weeks and does not include administration of any interferon or ribavirin. The DAAs can be administered at the same or different dosing frequencies. The patient being treated can be a treatment naive patient; a treatment experienced patient, including, but not limited to, a relapser, an interferon partial responder, an interferon non-responder, or a null responder; or a patient unable to take interferon. The patient may be infected with, for example and without limitation, HCV genotype 1, such as HCV genotype 1a or HCV genotype 1b; or HCV genotype 2 or 3; or HCV genotype 4, 5 or 6. The treatment according to this aspect of the technology may also be effective against other HCV genotypes. The DAAs can be administered around the same time or at different times. In addition to Compound 1 (or a salt thereof) and Compound 2 (or a salt thereof), said at least two DAAs can also include one or more additional DAAs selected from, for example, HCV protease inhibitors, HCV polymerase inhibitors, or HCV NS5A inhibitors. Non-limiting examples of such additional DAAs include PSI-7977, PSI-938, TMC-435, BMS-790052, BMS-650032, GS-5885, GS-9190, GS-9451, BI-201335, BI-207127, telaprevir, VX-222, mericitabine, and danoprevir.

In each aspect, embodiment, example or method described herein, Compound 1 (or a pharmaceutically acceptable salt thereof) can be administered, for example and without limitation, from 100 mg to 600 mg once daily, and Compound 2 (or a pharmaceutically acceptable salt thereof) can be administered, for example and without limitation, from 50 to 500 mg once daily. More preferably, Compound 1 (or a pharmaceutically acceptable salt thereof) is administered from 200 mg to 600 mg once daily, and Compound 2 (or a pharmaceutically acceptable salt thereof) is administered from 100 to 500 mg once daily. Highly preferably, Compound 1 (or a pharmaceutically acceptable salt thereof) is administered from 400 mg to 600 mg once daily, and Compound 2 (or a pharmaceutically acceptable salt thereof) is administered from 100 to 500 mg once daily. Preferably, Compound 1 (or a pharmaceutically acceptable salt thereof) can be administered 400 mg once daily, and Compound 2 (or a pharmaceutically acceptable salt thereof) is administered 120 mg once daily. Also preferably, Compound 1 (or a pharmaceutically acceptable salt thereof) can be administered 400 mg once daily, and Compound 2 (or a pharmaceutically acceptable salt thereof) can be administered 240 mg once daily.

A method of the present invention can be used to treat a naive patient or a treatment experienced patient. Treatment experienced patients include interferon non-responders (e.g., null responders), partial responders, and relapsers. A method of the present invention can also be used to treat patients who are not candidates for interferon treatment. Patients who are not candidates for interferon treatment include, but are not limited to, one or more of the following groups: patients intolerant to interferon, patients who refuse to take interferon treatment, patients with medical conditions which preclude them from taking interferon, and patients who have an increased risk of side effects or infection by taking interferon.

In any method described herein, one or more additional DAAs can be optionally used in the treatment regimen in addition to Compound 1 (or a salt thereof) and Compound 2 (or a salt thereof). These additional DAAs can be HCV protease inhibitors, HCV nucleoside or nucleotide polymerase inhibitors, HCV non-nucleoside polymerase inhibitors, HCV NS3B inhibitors, HCV NS4A inhibitors, HCV NS5A inhibitors, HCV NS5B inhibitors, HCV entry inhibitors, cyclophilin inhibitors, or combinations thereof.

Preferred HCV protease inhibitors for this purpose include, but are not limited to, telaprevir (Vertex), boceprevir (Merck), BI-201335 (Boehringer Ingelheim), GS-9451 (Gilead), and BMS-650032 (BMS). Other suitable protease inhibitors include, but are not limited to, ACH-1095 (Achillion), ACH-1625 (Achillion), ACH-2684 (Achillion), AVL-181 (Avila), AVL-192 (Avila), BMS-650032 (BMS), danoprevir (RG7227/ITMN-191, Roche), GS-9132 (Gilead), GS-9256 (Gilead), IDX-136 (Idenix), IDX-316 (Idenix), IDX-320 (Idenix), MK-5172 (Merck), narlaprevir (Schering-Plough Corp), PHX-1766 (Phenomix), TMC-435 (Tibotec), vaniprevir (MK-7009, Merck), VBY708 (Virobay), VX-500 (Vertex), VX-813 (Vertex), VX-985 (Vertex), or a combination thereof.

Preferred non-nucleoside HCV polymerase inhibitors for use in the present invention include, but are not limited to, GS-9190 (Gilead), BI-207127 (Boehringer Ingelheim), and VX-222 (VCH-222) (Vertex & ViraChem). Preferred nucleotide HCV polymerase inhibitors include, but are not limited to, PSI-7977 (Gilead), and PSI-938 (Gilead). Other suitable and non-limiting examples of suitable HCV polymerase inhibitors include ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir, TMC-647055 (Tibotec), VCH-759 (Vertex & ViraChem), VCH-916 (ViraChem), VX-759 (Vertex), GS-6620 (Gilead), IDX-102 (Idenix), IDX-184 (Idenix), INX-189 (Inhibitex), MK-0608 (Merck), RG7128 (Roche), TMC64912 (Medivir), GSK625433 (GlaxoSmithKline), BCX-4678 (BioCryst), ALS-2200 (Alios BioPharma/Vertex), ALS-2158 (Alios BioPharma/Vertex), or a combination thereof. A polymerase inhibitor may be a nucleoside or nucleotide polymerase inhibitor, such as GS-6620 (Gilead), IDX-102 (Idenix), IDX-184 (Idenix), INX-189 (Inhibitex), MK-0608 (Merck), PSI-7977 (Gilead), PSI-938 (Gilead), RG7128 (Roche), TMC64912 (Medivir), ALS-2200 (Alios BioPharma/Vertex), ALS-2158 (Alios BioPharma/Vertex), or a combination therefore. A polymerase inhibitor may also be a non-nucleoside polymerase inhibitor, such as PF-00868554 (Pfizer), ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir (Gilead),, TMC-647055 (Tibotec), VCH-759 (Vertex & ViraChem), VCH-916 (ViraChem), VX-222 (VCH-222) (Vertex & ViraChem), VX-759 (Vertex), or a combination thereof.

Preferred NS5A inhibitors include, but are not limited to, BMS-790052 (BMS) and GS-5885 (Gilead). Non-limiting examples of suitable NS5A inhibitors include GSK62336805 (GlaxoSmithKline), ACH-2928 (Achillion), AZD2836 (Astra-Zeneca), AZD7295 (Astra-Zeneca), BMS-790052 (BMS), BMS-824393 (BMS), GS-5885 (Gilead), PPI-1301 (Presidio), PPI-461 (Presidio) A-831 (Arrow Therapeutics), A-689 (Arrow Therapeutics) or a combination thereof.

Non-limiting examples of suitable cyclophilin inhibitors include alisporovir (Novartis & Debiopharm), NM-811 (Novartis), SCY-635 (Scynexis), or a combination thereof.

Non-limiting examples of suitable HCV entry inhibitors include ITX-4520 (iTherx), ITX-5061 (iTherx), or a combination thereof.

Specific examples of other DAA agents that are suitable for inclusion in a method of the present invention include, but are not limited to, AP-H005, A-831 (Arrow Therapeutics) (NS5A inhibitor), A-689 (Arrow Therapeutics) (NS5A inhibitor), INX08189 (Inhibitex) (polymerase inhibitor), ITMN-191 (Intermune/Roche) (NS3/4A Protease inhibitor), VBY-376 (Protease Inhibitor) (Virobay), ACH-1625 (Achillion, Protease inhibitor), IDX136 (Idenix, Protease Inhibitor), IDX316 (Idenix, Protease inhibitor), VX-813 (Vertex), SCH 900518 (Schering-Plough), TMC-435 (Tibotec), ITMN-191 (Intermune, Roche), MK-7009 (Merck), IDX-PI (Novartis), R7128 (Roche), PF-868554 (Pfizer) (non-nucleoside polymerase inhibitor), PF-4878691 (Pfizer), IDX-184 (Idenix), IDX-375 (Idenix, NS5B polymerase inhibitor), PPI-461 (Presidio), BILB-1941 (Boehringer Ingelheim), GS-9190 (Gilead), BMS-790052 (BMS), CTS-1027 (Conatus), GS-9620 (Gilead), PF-4878691 (Pfizer), RO5303253 (Roche), ALS-2200 (Alios BioPharma/Vertex), ALS-2158 (Alios BioPharma/Vertex), GSK62336805 (GlaxoSmithKline), or any combinations thereof.

The chemical structures of some of these optional HCV inhibitors are provided below:

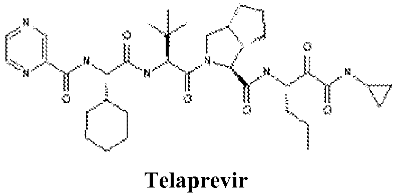

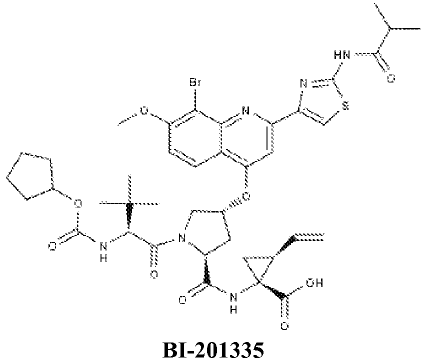

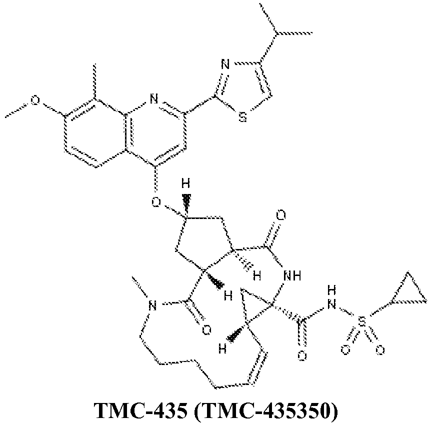

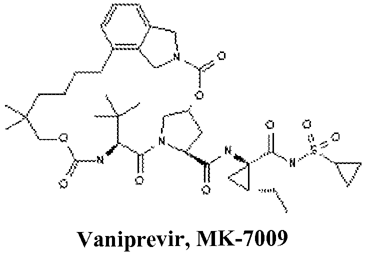

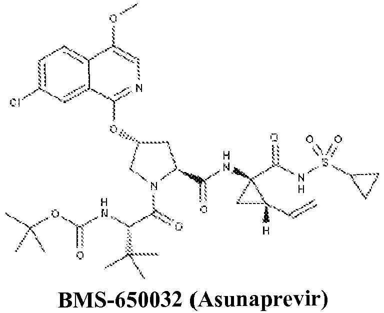

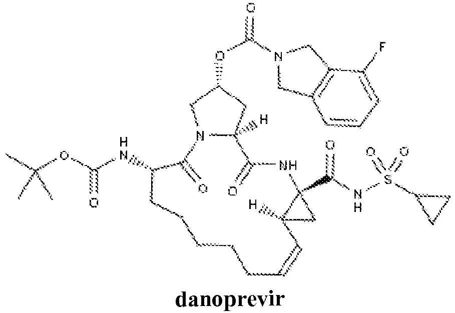

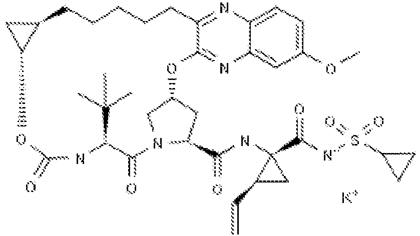

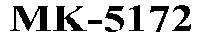

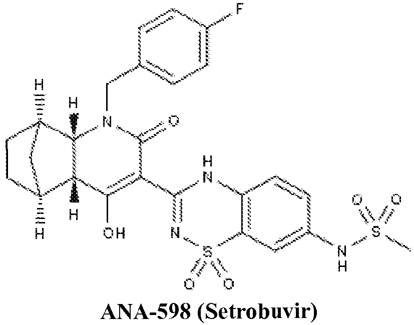

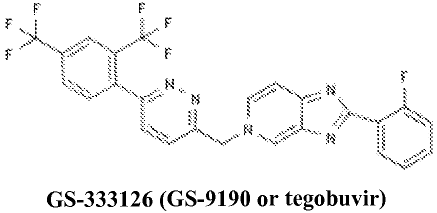

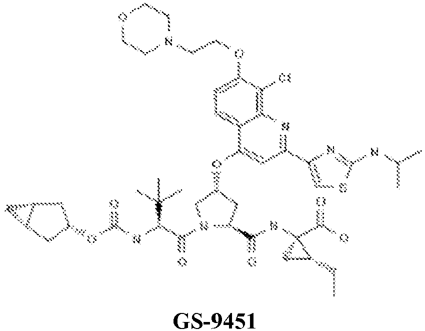

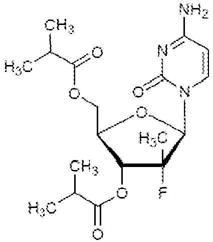

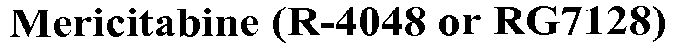

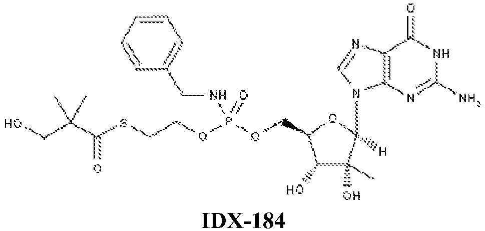

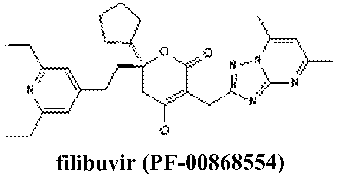

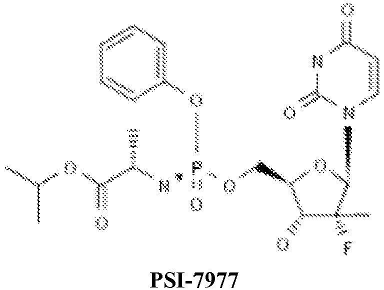

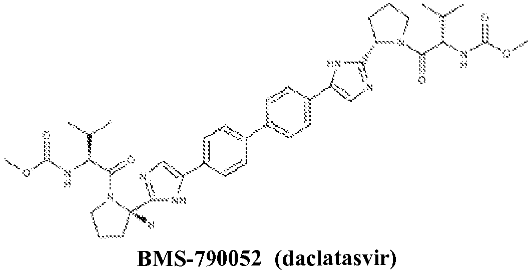

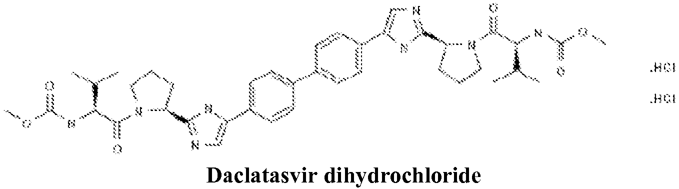

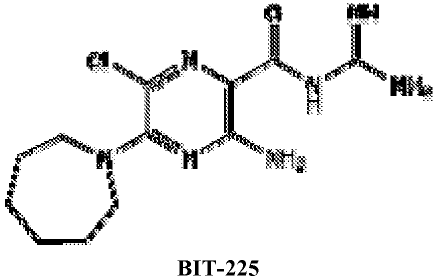

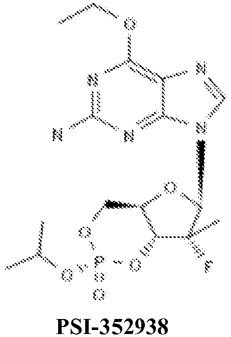

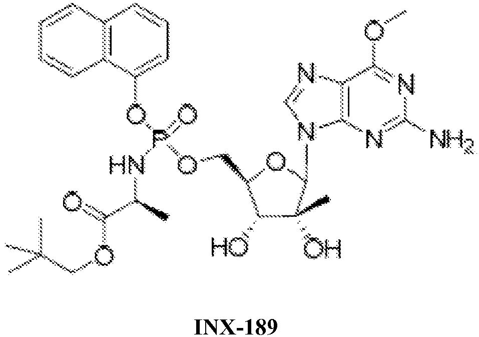

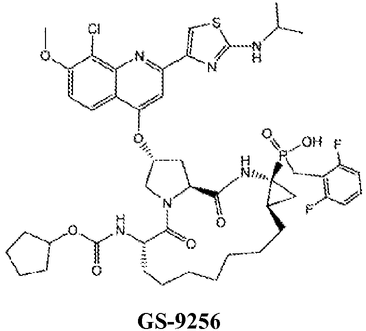

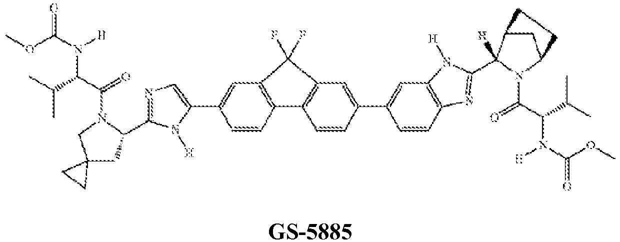

Any HCV inhibitor or DAA described herein encompasses its suitable salt forms when it is used in therapeutic treatments or pharmaceutical formulations.

In some embodiments, the present invention features methods for treating patients infected with HCV genotype 1, such as 1a or 1b. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naïve patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

In some embodiments, the present invention features methods for treating patients with HCV genotype 2 or 3 infection. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

In some embodiments, the present invention features methods for treating patients with HCV genotype 2 infection. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

In some embodiments, the present invention features methods for treating patients with HCV genotype 3 infection. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

In some embodiments, the present invention features methods for treating patients with HCV genotype 4 infection. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

In some embodiments, the present invention features methods for treating patients with HCV genotype 5 infection. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

In some embodiments, the present invention features methods for treating patients with HCV genotype 6 infection. The methods comprise administering to such a patient a combination of at least 2 DAAs for no more than 12 weeks (e.g., the duration being 12 weeks), such as no more than 8 weeks (e.g., the duration being 8 weeks), wherein the treatment does not include administration of either interferon or ribavirin, and said at least 2 DAAs comprise Compound 1 (or a pharmaceutically acceptable salt thereof) and Compound 2 (a pharmaceutically acceptable salt thereof). Compound 1 (or a pharmaceutically acceptable salt thereof) and Compound 2 (a pharmaceutically acceptable salt thereof) can be administered in therapeutically effective amounts to provide a SVR (for example, SVR12 or SVR24) after the completion of the treatment. The patients may be treatment naive patients or treatment experienced patients. The treatment duration can be no more than 12 weeks, including but not limited to, no more than 11 weeks, no more than 10 weeks, no more than 9 weeks, but preferably no more than 8 weeks, no more than 7 weeks, no more than 6 weeks, no more than 5 weeks, no more than 4 weeks, or no more than 3 weeks, e.g., the duration being 12 weeks, or the duration being 8 weeks.

It will be understood that the specific dose level for any particular patient will depend upon a variety of factors including the activity of the specific compound employed, the age, body weight, general health, sex, diet, time of administration, route of administration, rate of excretion, drug combination, and the severity of the disease undergoing therapy.

In any method described herein, Compound 1 (or a pharmaceutically acceptable salt thereof) and Compound 2 (a pharmaceutically acceptable salt thereof) may be co-formulated in a single dosage form. Non-limiting examples of suitable dosage forms include liquid or solid dosage forms. Preferably, Compound 1 and Compound 2 are formulated in a single solid dosage form in which at least one of the DAAs is in an amorphous form, or highly preferably molecularly dispersed, in a matrix which comprises a pharmaceutically acceptable water-soluble polymer and a pharmaceutically acceptable surfactant. The other DAAs can also be in an amorphous form or molecularly dispersed in the matrix, or formulated in different form(s) (e.g., in a crystalline form). More preferably, each of the two DAAs is in an amorphous form, or highly preferably molecularly dispersed, in a matrix which comprises a pharmaceutically acceptable water-soluble polymer and a pharmaceutically acceptable surfactant.

In any method described herein, the patient being treated can be a treatment-naive patient.

In any method described herein, the patient being treated can be an interferon non-responder.

In any method described herein, the patient being treated can be an interferon null-responder.

In any method described herein, the patient being treated can be without cirrhosis.

In any method described herein, the patient being treated can be a cirrhotic patient.

In any method described herein, the patient being treated can be a patient with compensated cirrhosis.

It should be understood that the above-described embodiments and the following examples are given by way of illustration, not limitation. Various changes and modifications within the scope of the present invention will become apparent to those skilled in the art from the present description.

### Example 1. Clinical Modeling for Interferon-free DAA Combination Therapies

Treatment regimens comprising administration of Compound 1 and Compound 2 were evaluated using clinical models described in U.S. Patent Application Publication No. 2013/0102526, filed October 19, 2012 and entitled "Methods for Treating HCV", which is incorporated herein by reference in its entirety. These treatment regimens comprised administration of Compound 1 and Compound 2, but did not include administration of either interferon or ribavirin.

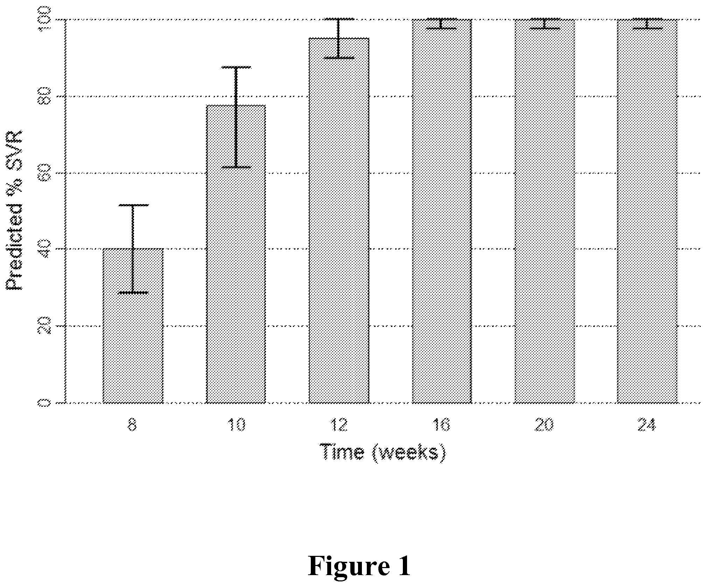

Figure 1 shows the predicted median SVR percentages and 90% SVR confidence intervals for 2-DAA regimens consisting of the use of Compound 1 (400 mg once daily) and Compound 2 (120 mg once daily) to treat genotype 1 naïve subjects. Different treatment durations were assessed. The predicted SVR rate for a 12-week treatment was about 95%. As used in all of the figures of the present application, the vertical bar at the top of each SVR percentage column represents the 90% SVR confidence interval, and the x-axis ("Time (weeks)") indicates the duration of each treatment regimen.

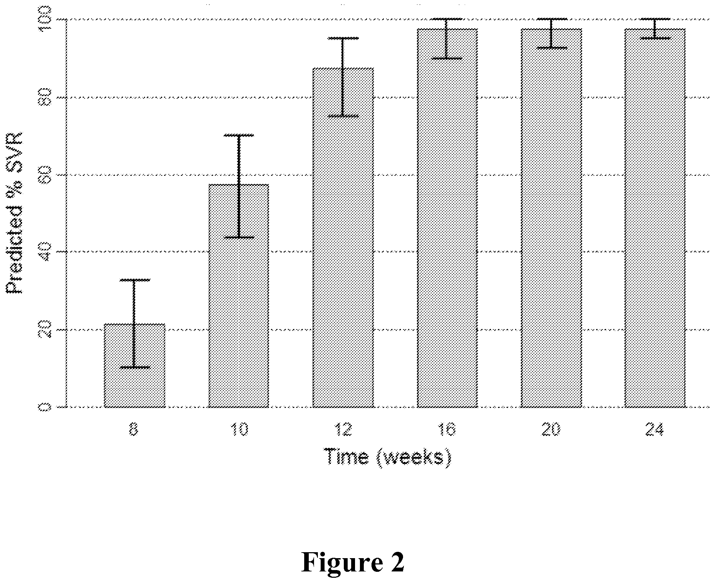

Figure 2 illustrates the predicted median SVR percentages and 90% SVR confidence intervals for 2-DAA regimens consisting of the use of Compound 1 (400 mg once daily) and Compound 2 (60 mg once daily) to treat genotype 1 naïve subjects. Different treatment durations were assessed. The predicted SVR rate for a 12-week treatment was about 85-90%.

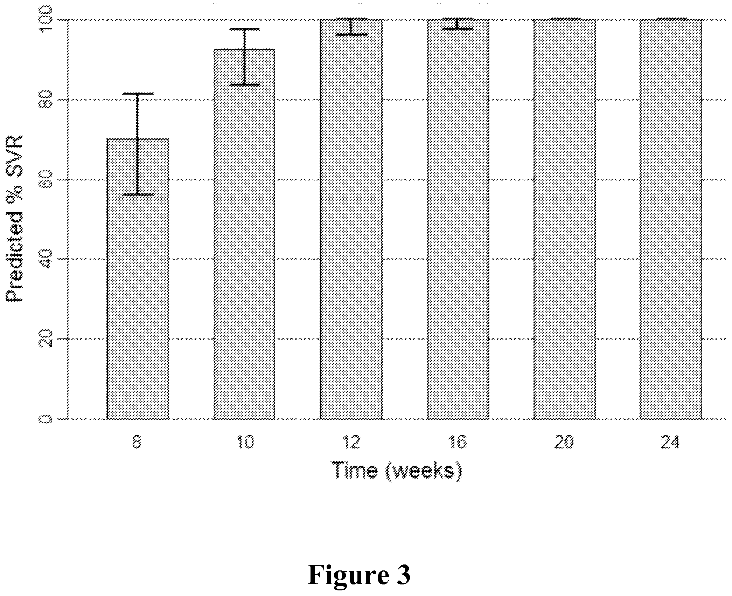

Figure 3 shows the predicted median SVR percentages and 90% SVR confidence intervals for 2-DAA regimens consisting of the use of Compound 1 (600 mg once daily) and Compound 2 (480 mg once daily) to treat genotype 1 naïve subjects. Different treatment durations were assessed. The predicted SVR rate for a 12-week treatment was about 100%.

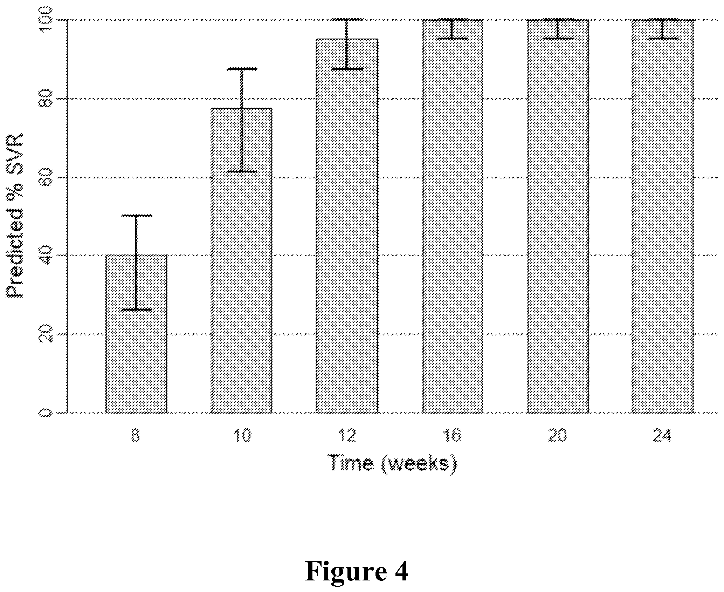

Figure 4 depicts the predicted median SVR percentages and 90% SVR confidence intervals for 2-DAA regimen consisting of the use of Compound 1 (400 mg once daily) and Compound 2 (120 mg once daily) to treat genotype 3 naïve subjects. Different treatment durations were assessed. The predicted SVR rate for a 12-week treatment was about 95%.

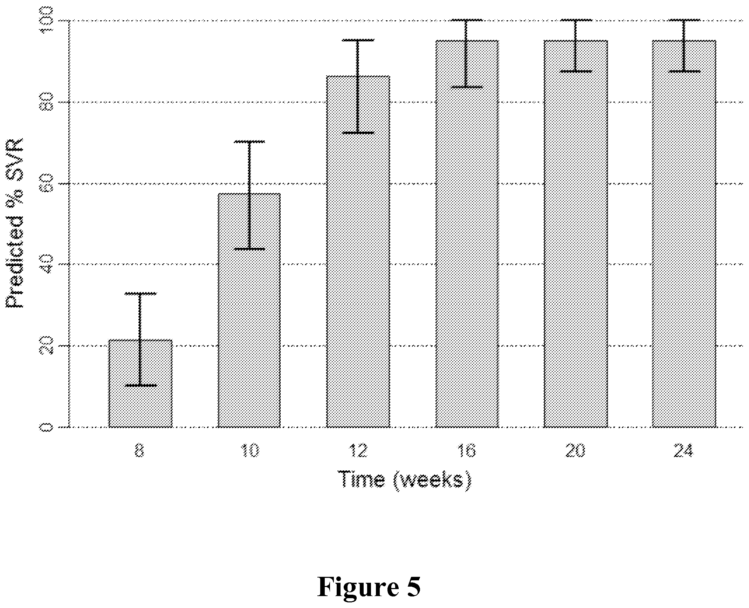

Figure 5 illustrates the predicted median SVR percentages and 90% SVR confidence intervals for 2-DAA regimen consisting of the use of Compound 1 (400 mg once daily) and Compound 2 (60 mg once daily) to treat genotype 3 naïve subjects. Different treatment durations were assessed. The predicted SVR rate of a 12-week treatment was about 85-90%.

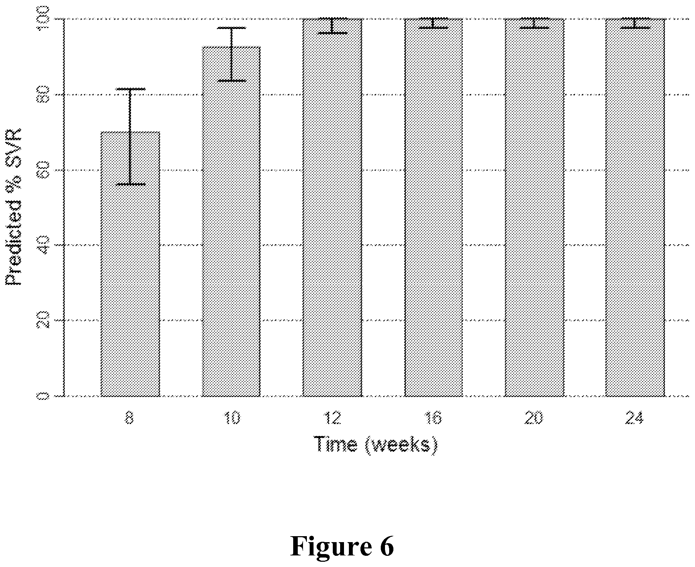

Figure 6 shows the predicted median SVR percentages and 90% SVR confidence intervals for 2-DAA regimens consisting of the use of Compound 1 (600 mg once daily) and Compound 2 (480 mg once daily) to treat genotype 3 naïve subjects. Different treatment durations were assessed. The predicted SVR rate of a 12-week treatment was about 100%.

### Example 2. Combination of Compound 1 and Compound 2 In Vitro

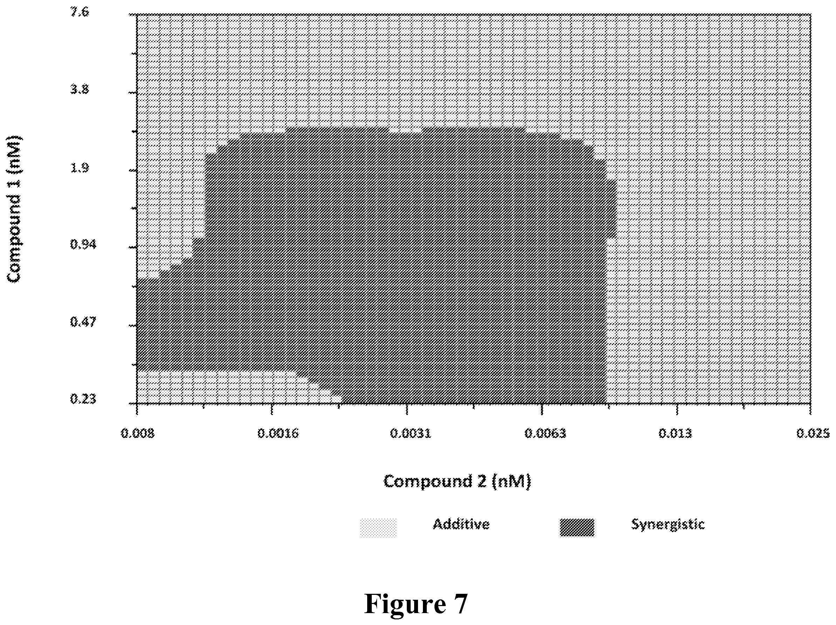

Figure 7 shows that the combination of Compound 1 and Compound 2 exhibits significant synergistic effect on HCV inhibition as tested in HCV GT 1b Con-1 replication cells. The result was generated using Prichard and Shipman model (Prichard et al. ANTIVIRAL RESEARCH 14:181-205 (1990)).

Compound 1 inhibited replication of HCV stable subgenomic replicons containing NS3 genes from GT 1a, 1b, 2a, 3a, 4a, or 6a with EC₅₀ values ranging from 0.85 to 2.8 nM. Of note, Compound 1 was potent against replicon containing GT3a protease, with an EC₅₀ value of 1.6 nM. Compound 1 retained its activity against common GT1a and 1b variants at NS3 amino acid positions 155 and 168 that conferred resistance to other HCV protease inhibitors (Pis). Resistant colony selection studies in GT1a and 1b subgenomic replicon cells identified A156T in GT1a and A156V in GT1b as the most frequent variants, which conferred 1400- and 1800-fold reduced susceptibility to Compound 1, respectively. However, these variants had *in vitro* replication capacities of only 1.5% and 9.2% that of their corresponding wild-type replicons. In a replicon containing GT3a NS3 protease, Compound 1 selected very few colonies at concentrations ≥ 100-fold over its EC50 value. The colonies that survived the selection contained either A156G alone, or Q168R co-selected with Y56H, which conferred 1500- or 1100-fold loss in susceptibility to Compound 1, respectively.

**Table 2. Antiviral Activity of Compound 1 in the HCV Subgenomic Stable Replicon Cell Culture Assay**

| **HCV Replicon Subtype** | **N^{b}** | **0% Human Plasma^{a} Mean EC₅₀, nM, ± Std. Dev.** |
|---|---|---|
| Genotype 1a | 9 | 0.85 ± 0.15 |
| Genotype 1b | 8 | 0.94 ± 0.35 |
| Genotype 2a | 2 | 2.7 ± 1.1 |
| Genotype 3a | 2 | 1.6 ± 0.49 |
| Genotype 4a | 4 | 2.8 ± 0.41 |
| Genotype 6a | 4 | 0.86 ± 0.11 |

| | | |
|---|---|---|
| a. The 0% human plasma assay contains 5% fetal bovine serum b. Number of independent replicates | | |

**Table 3. Antiviral Activity of Compound 1 in the HCV Subgenomic Stable Replicon Cell Culture Assay**

| **HCV Replicon Subtype** | **N^{b}** | **40% Human Plasma^{a} Mean EC₅₀, nM, ± Std. Dev.** |
|---|---|---|
| Genotype 1a | 10 | 5.3 ± 1.0 |
| Genotype 1b | 8 | 10 ± 5.0 |

| | | |
|---|---|---|
| a. The 0% human plasma assay contains 5% fetal bovine serum b. Number of independent replicates | | |

When tested against common HCV genotype 1 NS3 resistance-associated variants, such as V36M, R155K, D168A and D168V in GT 1a (H77), or T54A, R155K, D168V and V170A in GT 1b (Con-1), Compound 1 showed inhibitory activity nearly equivalent to that against wild-type HCV replicon. Compound 1 was also shown to have potent activity against many NS5A inhibitor and NS5B inhibitor resistance-associated variants *in vitro* (e.g., M28T, M28V, Q30D, Q30R, Y93C, Y93H, Y93N, L31V+Y93H, C316Y, M414T, Y448C, Y448H, S556G and S559G in GT 1a, and L28T, Y93H, S282T, C316Y, Y448H and S556G in GT 1b).

The foregoing description of the present invention provides illustration and description, but is not intended to be exhaustive or to limit the invention to the precise one disclosed. Modifications and variations are possible in light of the above teachings or may be acquired from practice of the invention. Thus, it is noted that the scope of the invention is defined by the claims and their equivalents.
The following numbered paragraphs contain statements of broad combinations of the inventive technical features herein disclosed.
1. A method for treatment for HCV, comprising administering at least two direct acting antiviral agents (DAAs) to an HCV patient, wherein neither interferon nor ribavirin are administered to said patient during said treatment, and said treatment lasts for 8, 9, 10, 11 or 12 weeks, and wherein said at least two DAAs comprise:
   Compound 1 or a pharmaceutically acceptable salt thereof, and
   Compound 2 or a pharmaceutically acceptable salt thereof.
2. The method of paragraph 1, wherein said treatment lasts for 12 weeks.
3. The method of paragraph 1, wherein said patient is infected with HCV genotype 1.
4. The method of paragraph 1, wherein said patient is infected with HCV genotype 1a.
5. The method of paragraph 1, wherein said patient is infected with HCV genotype 2.
6. The method of paragraph 1, wherein said patient is infected with HCV genotype 3.
7. The method of paragraph 1, wherein said patient is infected with HCV genotype 4.
8. The method of paragraph 1, wherein said patient is infected with HCV genotype 5.
9. The method of paragraph 1, wherein said patient is infected with HCV genotype 6.
10. The method of paragraph 2, wherein said patient is infected with HCV genotype 1.
11. The method of paragraph 2, wherein said patient is infected with HCV genotype 1a.
12. The method of paragraph 2, wherein said patient is infected with HCV genotype 2.
13. The method of paragraph 2, wherein said patient is infected with HCV genotype 3.
14. The method of paragraph 2, wherein said patient is infected with HCV genotype 4.
15. The method of paragraph 2, wherein said patient is infected with HCV genotype 5.
16. The method of paragraph 2, wherein said patient is infected with HCV genotype 6.
17. The method of paragraph 1, wherein said patient is without cirrhosis.
18. The method of paragraph 1, wherein said patient is with compensated cirrhosis.
19. The method of paragraph 2, wherein said patient is without cirrhosis.
20. The method of paragraph 2, wherein said patient is with compensated cirrhosis.
21. The method of paragraph 1, wherein said patient is a treatment-naive patient.
22. The method of paragraph 1, wherein said patient is an interferon non-responder.
23. The method of paragraph 2, wherein said patient is a treatment-naive patient.
24. The method of paragraph 2, wherein said patient is an interferon non-responder.

## Claims

1. Two direct acting antiviral agents (DAAs) for use in a method of treatment for hepatitis C virus (HCV), wherein said two DAAs comprise or consist of: 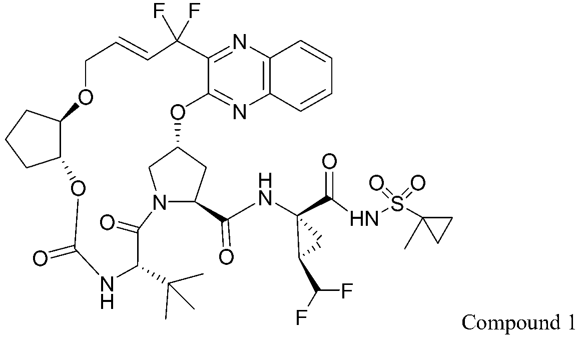
or a pharmaceutically acceptable salt thereof, and 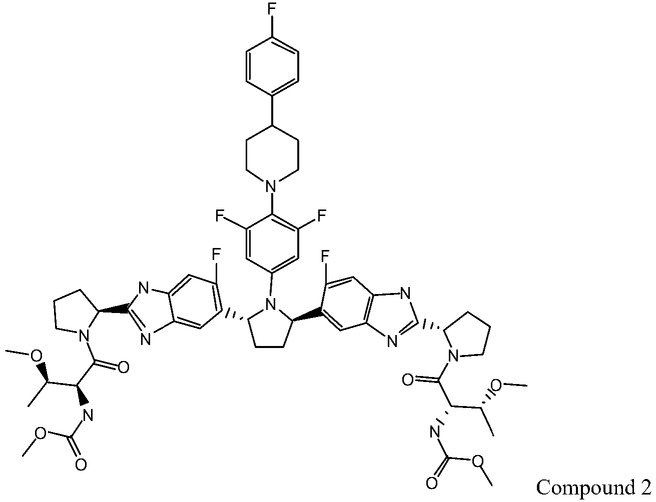
or a pharmaceutically acceptable salt thereof,
and wherein neither interferon nor ribavirin are administered to said patient during said treatment, and said treatment lasts for 8, 12, or 16 weeks.

2. At least two direct acting antiviral agents (DAAs) for use in a method of treatment for hepatitis C virus (HCV), wherein said at least two DAAs comprise of: 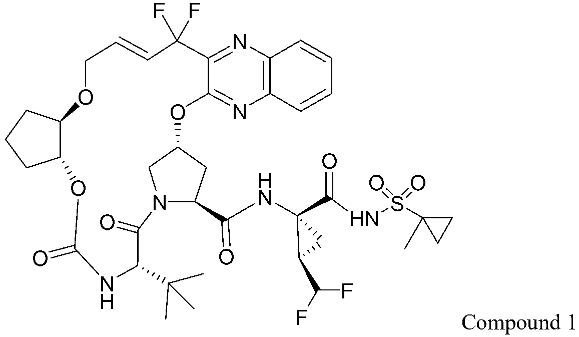
or a pharmaceutically acceptable salt thereof, 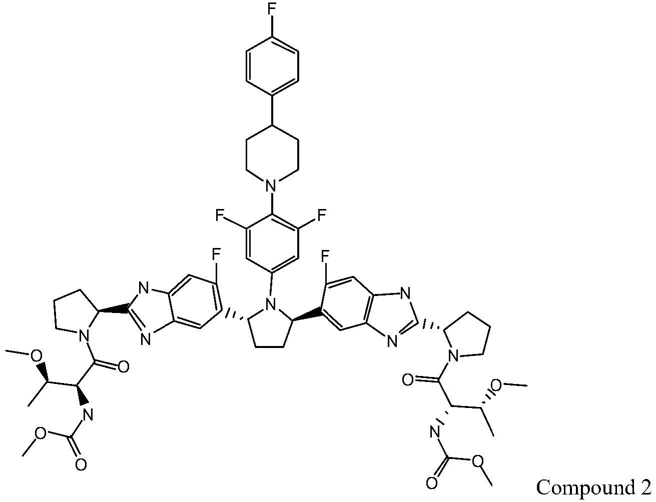
or a pharmaceutically acceptable salt thereof,
and another anti-HCV agent;
and wherein neither interferon nor ribavirin are administered to said patient during said treatment, and said treatment lasts for 8, 12, or 16 weeks;
optionally wherein the anti-HCV agent is selected from protease inhibitors, nucleoside or nucleotide polymerase inhibitors, non-nucleoside polymerase inhibitors, NS3B inhibitors, NS4A inhibitors, NS5A inhibitors, NS5B inhibitors, cyclophilin inhibitors, or combinations thereof.

3. The two DAAs for use according to claim 1 or 2, wherein said treatment lasts for 8 weeks.

4. The two DAAs for use according to claim 1 or 2, wherein said treatment lasts for 12 weeks.

5. The two DAAs for use according to claim 1 or 2, wherein said treatment lasts for 16 weeks.

6. The two DAAs for use according to any one of claims 1 to 5, wherein said patient is infected with HCV genotype 1.

7. The two DAAs for use according to any one of claims 1 to 5, wherein said patient is infected with HCV genotype 1a.

8. The two DAAs for use according to any one of claims 1 to 5, wherein said patient is infected with HCV genotype 1b.

9. The two DAAs for use according to any one of claims 1 to 5, wherein said patient is infected with HCV genotype 2.

10. The two DAAs for use according to any one of claims 1 to 5, wherein said patient is infected with HCV genotype 3.

11. The two DAAs for use according to any one of claims 1 to 5, wherein said patient is infected with HCV genotype 4.

12. The two DAAs for use according to any one of claims 1 to 5, wherein said patient is infected with HCV genotype 5.

13. The two DAAs for use according to any one of claims 1 to 5, wherein said patient is infected with HCV genotype 6.

14. The two DAAs for use according to any one of claims 1 to 13, wherein said patient is also:
(i) without cirrhosis;
(ii) a cirrhotic patient;
(iii) with compensated cirrhosis;
(iv) a treatment-naive patient;
(v) a treatment-experienced patient;
(vi) a relapser;
(vii) an interferon partial responder;
(viii) an interferon non-responder;
(ix) an interferon null-responder; or
(x) a patient unable to take interferon.

15. The two DAAs for use according to any one of claims 1 to 14, wherein Compound 1 or a pharmaceutically acceptable salt thereof, and Compound 2 or a pharmaceutically acceptable salt thereof, are administered once daily to said patient;
optionally wherein said treatment comprises administering 200 mg to 600 mg Compound 1 once daily and 100 mg to 500 mg Compound 2 once daily to said patient.
